# EUROPEAN PATENT APPLICATION

(11) **EP 3 842 029 A1**
(43) Date of publication of application: **30.06.2021**
(21) Application number: 18930577.4
(22) Date of filing: 21.08.2018
(51) Int. Cl.: A61K 9/00, A61K 31/4168, A61K 31/541, A61K 47/44, A61P 33/02, A61P 35/00

(54) **NANOSTRUCTURED NANOPARTICLES COMPRISING ONE OR MORE ACTIVE INGREDIENTS FOR THE TREATMENT OF DISEASES CAUSED BY TRYPANOSOMES AND FOR THE TREATMENT OF TUMOURS OF NEURAL ORIGIN, COMPOSITIONS COMPRISING SAME, A PREPARATION METHOD AND THERAPEUTIC USE THEREOF**

(71) Applicant: Gador Limitada, Providencia, Santiago (CL)
(72) Inventor: CARRICARTE, Valentina, Providencia, Santiago (CL); ROLDAN, Emilio, Providencia, Santiago (CL)
(74) Representative: Pons
(86) International application number: PCT/CL2018/050067
(87) International publication number: WO 2020/037438

(57) **Abstract**

The invention related to a pharmaceutical composition with antineoplastic and antitumor action, and a method for obtaining same, wherein said composition comprises structured lipid nanoparticles that have a diameter of 0.1 nm to 100 nm, are spheroidal in shape and contain particles of one or more active ingredients for the treatment of diseases caused by trypanosomes and for the treatment of tumors of neural origin, and at least one coupling of two fatty acids formed by a saturated fatty acid or fat that is semi-solid at a temperature of 19ºC to 21ºC and an unsaturated fatty acid that is liquid at a temperature of 19ºC to 21ºC and in turn the method for obtaining same finally contains the nanoparticles of one or more active ingredients for trypanosomiasis in colloidal suspension, the zeta potential ranges from -30 to +30, preferably +8, the particles measured by zetasizer and ultramicroscopy have a diameter of 0.1 nanometers to 100 nanometers, preferably 1 nanometer in diameter, and a spheroidal shape.

## Description

### 1) TECHNICAL FIELD OF THE INVENTION

The present invention refers to a "Pharmaceutical composition with antineoplastic and antitumor action and method of obtaining it", applied in the field of diseases caused by trypanosomes, preferably for the treatment of Chagas Mazza disease and for the treatment of tumors of neural origin, of the central nervous system such as astrocytoma, retinoblastoma and neuroblastoma.

### 2) STATE OF THE ART AND PROBLEMS DETECTED

Chagas disease is currently endemic in Latin America and is a major public health problem in the entire region. It is estimated that this disease affects approximately 15 to 20 million people, from southern California to Argentina and that more than 100 million individuals would be exposed to the risk of contracting the infection. Thus, it is estimated that between 2 and 3 percent of the population of Latin America would be infected. The morbidity and mortality of Chagas are ten times higher than those exhibited by other parasitic diseases in the subcontinent [1, 2, 3, 4]. The causative agent of Chagas disease is the flagellated protozoan Trypanosoma cruzi, which is transmitted to humans through the bite of the vinchuca (Triatoma infestans) and other blood-sucking insects of the Reduviidae family [5]. In most cases (> 80%), human infection is related to insect feces, blood transfusion, or organ transplant (15%) [6]. The finding of autochthonous transmission makes Chagas a potentially emerging disease in the United States [7]. Furthermore, Latin American emigration has brought this disease to developed countries in Europe and Oceania [8].

The biological cycle of the parasite is mainly composed of three fundamental cell forms:
a) trypomastigotes, which are found in the blood of mammals, spreading the infestation transmitted from the intestine of the hematophagus;
b) epimastigotes, which is the proliferative form present in the intestine of the insect; and
c) amastigotes, which multiply through binary fission within mammalian host cells, producing their lysis and releasing new trypomastigotes into the bloodstream. (See
Functional effectiveness of nifurtimox nanoparticles in the in vitro treatment of chagas disease). In this way, they can invade any nucleated cell and start a new reproductive cycle [9].

The infection occurs in three phases: acute, undetermined and chronic. The acute phase of the disease is characterized by high parasitaemia, consisting of trypomastigotes. After a period ranging from a few weeks to several months, the infection is usually well controlled by the host's immune response. However, parasites continue their cycle in and out of mammalian cells and appear in the bloodstream only occasionally, making their detection difficult. Symptoms are often not evident for a long time (maybe decades), which is why this period is referred to as the undetermined phase. About 40% of infected patients end up developing the chronic phase of the disease, in which intracellular amastigotes cause irreversible structural damage to the esophagus, colon, and heart [2].

The first-line drugs authorized for the treatment of Chagas disease are nifurtimox (3-methyl-4- (5'-nitrofurfurylidene-amino) -tetrahydro-4H-1,4-thiazine-1,1-dioxide, Bayer 2502) and benznidazole (2-nitroimidazole (N-benzyl-2-nitroimidazole) acetamide, RO 7-1051). It has been suggested in different studies that ethanidazole, derived from nitroimidazole, eflornithine, melarsoprol, pentamidine, surimin, fexinidazole could also be used. These two drugs (nifurtimox and benznidazole) have been used for this purpose for decades [10] and, to date, they could not to be replaced, even though both have serious side effects and low efficacy [11-15]. Both drugs are effective in the acute phase, chronic infections in children up to 12 years of age, and in congenital infections [2], but have little or no activity during the chronic phase of the disease. In vitro, nifurtimox and benznidazole are able to kill the intracellular form of the parasite (amastigotes). Circulating parasites (trypomastigotes) are also killed by these agents in vivo, but no benefit is seen when these drugs are used in adult humans during the undeterined and chronic phases of the disease [19]. After intestinal absorption, these drugs easily reach the tissues, but high doses are required to eliminate circulating parasites. Thus, it has been proposed that an increase in the arrival of drugs to the affected tissues (reservoirs) could improve therapeutic efficacy [20].

To date, no effective vaccines or treatments are available on the market for adults with chronic Chagas disease [16]. This is mainly because Chagas belongs to the group of the so-called "neglected" diseases, which are endemic in low-income populations in developing regions of Africa, Asia, and the Americas [17]. The lack of better pharmacotherapy is due to the absence of effective public policies in the countries suffering from the endemic [18]. Private pharmaceutical companies have also shown no interest.

In 2007, the World Health Assembly (WHA) proclaimed the right of the child to have access to safe, effective and proven medicines. But the existing drugs as nifurtimox and benznidazole, exist only in solid pharmaceutical forms. High doses and complex administration schedules affect the lifestyle of young patients, and decreased adherence levels dramatically restrict treatment success [21].

Children can be cured through liquid formulations of nifurtimox or benznidazole, but these are not commercially available. For this reason, the liquid forms are called "orphan drugs". In this context, the officinal processing of original solid pharmaceutical forms [22], becomes the only alternative for the treatment of pediatric patients with the disease. However, due to the extreme insolubility of the active ingredient, the erratic bioavailability of their suspensions and the serious lack of resources in countries with fragile health systems and limited infrastructure, the quality, safety and efficacy of these extemporaneous formulations are doubtful [23-25].

For these reasons, there is an urgent need for new and effective anti-chagasic products. At present, there are mainly two possible strategies to carry out programs aimed at producing new drugs for the treatment of infected patients: i) promoting the search for new drug candidates or ii) developing improved drugs for the administration of already approved drugs available on the market [26].

In this sense, the design and development of new safe, effective and efficient antiparasitic drug delivery systems is essential. In particular, new systems are necessary for the release of nifurtimox, benznidazole, and the rest of those mentioned herein that allow a reduction of the dose to be administered, improving its absorption. Also, there is an unmet need to develop liquid formulations of nifurtimox and benznidazole, in particular, pharmaceutical forms to be used in pediatrics.

The preparation of conventional and stable pharmaceutical forms based on nifurtimox or benznidazole is complex, due to their physicochemical properties and, in particular, their low solubility in water. During the last years, nano-sized objects (nanoparticles, with a diameter of 0.1 nanometers to 1000 nanometers, preferably from 1 nanometer to 500 nanometers) have been proposed as vehicles for drugs that are difficult to use in terms of bioavailability, solubility, pharmacokinetics, etc. Liposomes, known for several decades, contain an internal cavity suitable for carrying out said transportation. However, the benznidazole-loaded liposomes did not show parasiticidal activity. In addition, unilamellar liposomes suffer from fragility, which is why they release the drug early, while multilamellar liposomes fail in the "delivery" of the active ingredient, as they are excessively resistant and do not release the drug. Until the presentation of the present patent, other attempts to use different nano objects have collided with the toxicity of the chosen vehicles [37].

Certain types of nano objects, solid lipid nanoparticles (SLN), have the following advantages when used for controlled drug delivery: high tissue tolerance, low regulatory requirements if physiologically acceptable lipids are used, the ability to transport lipophilic and hydrophilic drugs, protection against chemical degradation of the active ingredients, possibility of fixing the target and low production cost. However, they show some intrinsic limitations, such as their low transport capacity and a tendency to eject the drug during the storage period [33-35].

A variety of SLNs, called structured lipid nanoparticles (NLC mentioned above), have been shown to share the mentioned favorable characteristics, while not having the limitations described above.

In the same conceptual order, there are other forms of trypanosomiasis, for example, African Trypanosomiasis, also called sleeping sickness, which is caused by Trypanosoma brucei gambiensis. This parasite is also sensitive to nifurtimox and therefore the improvement of the pharmaceutical / pharmacological properties of nifurtimox should benefit the therapeutic regimens for this disease.

It is also known that nifurtimox has been used to mitigate the growth of the most common extracranial solid tumor in children, Neuroblastoma, which has a very poor prognosis. The mechanism of action of nifurtimox on in vitro neuroblastoma tumor cells has been shown to be the acceleration of apoptosis [38-39]. This mechanism of action on certain tumor cells of the host would allow its use in non-parasitic diseases. The inventors have tested, with very good results, nanoparticles of nifurtimox in in vitro cultures of Astrocytoma and, surprisingly in Retinoblastoma (see In vitro effectiveness of nanoparticles of nifurtimox on apoptosis of tumor cells of the central nervous system). In the case of Astrocytoma in spherical and adherent MSP12 cell lines, spherical and adherent MGG8 and GL26 NT (rat) and, in an unprecedented way, in the case of Retinoblastoma in the Y79 cell line. Consequently, the pharmaceutical-pharmacological application of nanoparticles of nifurtimox will benefit the usual therapeutic schemes for these diseases and / or will allow indications in tumor variants that today do not have rational treatments.

### 3) PROVIDED SOLUTION

Various types of particles are known to be used in drug delivery. Depending on their size, they can be classified into microparticles and nanoparticles.

The present inventors have developed novel structured lipid nanoparticles (NLC) that comprise particles of one or more active ingredients for the treatment of trypanosomiasis with at least one coupling of two fatty acids formed by a saturated fatty acid or a semisolid lipid. at the temperature between 19 degrees centigrade and 21 degrees centigrade, preferably at 20 degrees centigrade; and a liquid unsaturated fatty acid or a liquid lipid at the temperature of between 19 degrees centigrade and 21 degrees centigrade, preferably at 20 degrees centigrade; wherein said nanoparticles have a mean effective size of between 0.1 nm and 100 nm, preferably from 1 nanometer to 10 nanometers, particularly 1 nm, where the saturated fatty acid or semisolid lipid is selected from palmitic acid or stearic acid, and the unsaturated fatty acid or liquid lipid is selected from oleic acid, arachidonic acid, palmitoleic acid, alpha-linoleic acid, or gamma-linoleic acid. In order to improve the aqueous suspensibility of therapeutically active substances, decrease their toxicity, increase their efficacy, and prolong the stability of the pharmaceutical forms prepared with them, the present inventors have investigated the encapsulation of drugs within structured lipid nanoparticles (NLC) [27-32].

Thus, the inventors have developed a pharmaceutical composition that includes novel nano objects, useful in the preparation of stable liquid dosage forms of active compounds against trypanosomiasis, in particular nifurtimox and benznidazole, especially intended for their administration to pediatric patients.

From the highly ordered structure presented at room temperature by solid lipids such as stearic acid and palmitic acid, the incorporation of liquid lipids into the crystals of such solid lipids leads to a massive disruption of their organization. The resulting matrix of lipid particles shows large imperfections in the crystal lattice and leaves enough space to accommodate molecules of the active ingredient, which improves the drug-loading capacity of the nano-structured vehicle [32]. NLCs do not have the limitations associated with SLNs, such as small loading capacity and drug ejection during storage.

In conclusion, a pharmaceutical composition with novel nanostructured nanoparticles (NLC, Nano Lipidic Carriers) has been developed, comprising particles of one or more active ingredients for the treatment of trypanosomiasis and, at least, a coupling of two fatty acids formed by a saturated fatty acid or a semisolid lipid at a temperature between 19 degrees centigrade and 21 degrees centigrade, preferably 20 degrees centigrade; and a liquid monounsaturated fatty acid or a liquid lipid, at a temperature between 19 degrees centigrade and 21 degrees centigrade, preferably at 20 degrees centigrade; wherein said nanoparticles have a mean effective size of between 0.1 nm and 100 nm, preferably from 1 nanometer to 10 nanometers, where the saturated fatty acid or semisolid lipid is selected from stearic acid and palmitic acid, and the unsaturated fatty acid or liquid lipid it is selected from oleic acid, arachidonic acid, palmitoleic acid, alpha-linoleic acid, or gamma-linoleic acid. Thus, these novel nano objects are useful in the preparation of stable liquid dosage forms of active compounds against trypanosomiasis, in particular nifurtimox and benznidazole, especially intended for their administration to pediatric patients (acute intervention) and adult patients (chronic intervention) .

Likewise, NLCs carriers of selected active ingredients have been developed, in different pharmaceutical forms, with antineoplastic and antitumor action, especially effective for tumors of neural origin, preferably Retinoblastoma and Astrocytoma, both in vitro and in vivo.

The use of nanoparticles as drug carriers began with liposomes, which were developed by A. Bangham et al. in 1964. Its use as a micrometric drug carrier was tested by G Georgiadis et al. in 1971. The first nanometric tests belong to the same author in 1972. Structured lipid nanoparticles (NLC) were developed by Kawashima et al. In 1998, using the solvent diffusion method. The NLCs prepared based on the solvent diffusion method with oleic and stearic acids were developed by Wu et al. in 2004. The idea of using nanoparticles as carriers of anti-chagasic drugs (benznidazole loaded liposomes) was published by E Romero et al. in 2004. None of this could be patented. Below is a comparative table of the methods used by Hu et al. (2005) and the inventors of the present invention to prepare NLC through the solvent diffusion technique:

**Table 1 In this table, the technical similarities and differences and results can be observed. The volumes of water, acetone and alcohol, and the weight of fatty acids used are the same, as well as the working temperature. The technical differences reside in the pH used at the time of precipitation and the nanoparticle separation procedure.**

| **Autor** | **Hu *et al.*** | **CIN-Nanof** |
|---|---|---|
| Technique | **Diffusion of de solvents** | **Diffusion of solvents** |
| Particles | **NLC and SLN** | **NLC** |
| Drug (API) | **Clobetasol** | **Nifurtimox** |
| Preparation of empty nanoparticles | **Yes** | **no** |
| Proportion OA/SA | **Oleic/Stearic 30/70** | **Linoleic/Palmitic 30/70** |
| Approximate size of the obtained nanoparticles. | **150 to 300 nm** | **1 nm** |
| Solvent for the ingredients | **acetone/ethanol a/a** | **acetone/ethanol a/a** |
| Diffuser | **Distilled water** | **Distilled water** |
| Approximate temperatura during the preparation | **70°C** | **70°C** |
| Separation (1° part) | **Precipitation at pH= 1,2** | **Precipitation at pH= 2,0** |
| Separation (2° part) | **Centrifuging at 25.000 rpm** | **Filtered by paper** |
| Location of the nanoparticles | **In the centrifugation** | **In the supernatant** |
| % of API charge | **3,3 to 3,6 % w/w** | **3,5 a 4,7 % w/w** |
| % of incorporated API | **50 to 70 %** | **96-98 %** |
| Suspensibility in distilled water | **medium** | **Very high (178 mg/ml)** |
| Potential Z in mV | **-50** | **+8** |

A big difference is that, according to Hu et al., they would be found in the precipitate obtained by centrifugation, and instead in our technique they are found in the supernatant, where they can be visually detected by their intense yellow color, typical of the nifurtimox incorporated, in colloidal suspension, in a clear liquid. If we copied Hu's technique, we would find neither nanoparticles nor nifurtimox in the precipitate obtained by centrifugation, a test that we actually carried out. We confirmed this experimentally also by identification and naming of the precipitate obtained by filtration. Both precipitates are practically formed by fatty acids, without traces of nifurtimox.

Some of the advantages of NLC are observed in the last lines: the capture of the drug in the Hu preparation is quite good, up to 70%, but in the CIN-Nanof preparation it rises to almost all of the nifurtimox (it solves the problem of removing the drug not incorporated in the NLC from the liquid). The high suspensibility of the new NLCs is also very interesting, considering that it serves to pharmaco-technically solve the problem of the almost total insolubility of nifurtimox-drug in water when making pharmaceutical preparations.

**Table 2. Effects of pH in the 10 mg / 100ml suspension. The present table shows the wide pH range (from 2.5 to 9) in which the NLC suspension prepared by CIN-Nanof appears macroscopically stable.**

| pH | State |
|---|---|
| 1,7 | flocculate |
| 2 | Turbidity |
| 2,5 | Opalescense |
| 3 | Transparency |
| 4 | Transparency |
| 5 | Transparency |
| 6 | Transparency |
| 7 | Transparency |
| 8 | Transparency |
| 9 | Opalescense |
| 9,5 | Turbidity |
| 10 | Flocculate |

### Granulometric measurement of NLCs

The results of the measurement of the average size of the NLCs using the Z-Sizer are reproduced. The results can be seen in Table 3.

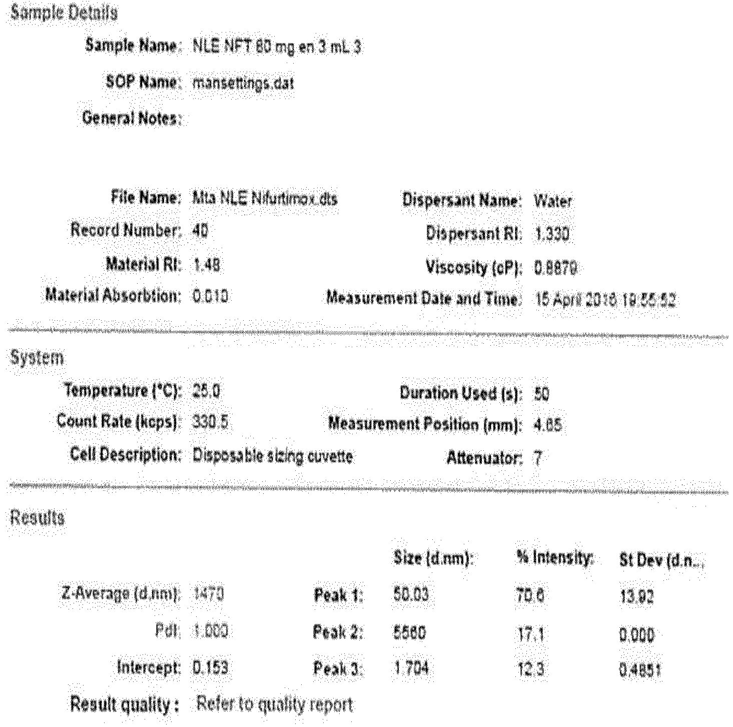

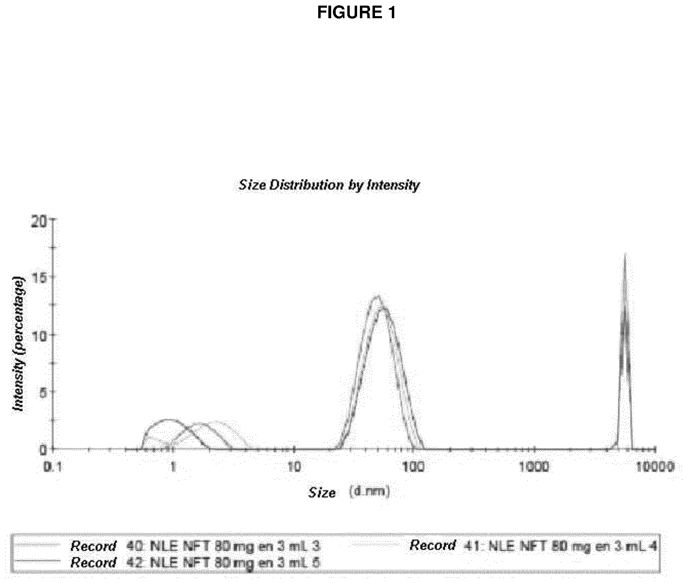

At the same time, in FIGURE 1, it can be seen that the NLCs carriers nifurtimox present a trimodal distribution at the average diameters of 1.7 nm, 50 nm and 5580 nm (the latter being obviously non-nanometric). However, subjecting the suspension to filtering through a 220 nm Teflon filter, the measurement becomes bimodal, with an average diameter distribution of 1.0 nm (very predominant) with a standard deviation of 0.05 nm., and a small part at the average diameter of 50 nm and standard deviation of 14.0 nm, the 5.6 micron mode having completely disappeared.

Meanwhile, there is no yellowish residue left in the filter, nor macroscopic lumps, the washing water circulating freely and effortlessly. The interpretation of this behavior is as follows: the size of the particles is 1 nm and the peaks of 50 and 5500 nm are produced by agglomerations of the same (probably generated thermodynamically due to the high surface / volume ratio of the nano-objects), which are crumbled by the mechanical effect of filtering. As confirmation of this hypothesis, if the filtered liquid is allowed to stand for 48 hours, agglomerates of approximately 50 and 5000 nm spontaneously form again, although in less quantity than in the original unfiltered suspension. Another confirmation lies in the direct observation of isolated NLCs (with a diameter of approximately 1 nm) and their agglomerates, through transmission electron microscopy and atomic force, the images of which are given below.

**Table 4: Compared solubility and suspensibility It is observed that the NLCs prepared by the inventors of the present invention of nifurtimox, are very suspensible in water, in comparison with the drug nifurtimox, favoring, as explained, the making of pharmaceutical preparations.**

| **product** | **Distilled water** | **Absolute ethanol** | **dimethilsulfoxide** | **acetone** | **chloroform** |
|---|---|---|---|---|---|
| **Nifurtimox propio** | insoluble | soluble | very soluble | very soluble | Little soluble |
| **Nifurtimox** | insoluble | Soluble | very soluble | very | Little soluble |
| **Sigma-Aldrich** | | | | soluble | |
| **Nanostructured Lipid Nanoparticles NLC** | **very suspensible** | Little soluble | soluble | very soluble | little soluble |

### □ Photomicrographs of the NLCs:

### □. Photomicrograph A) Transmission electron microscope (TEM).

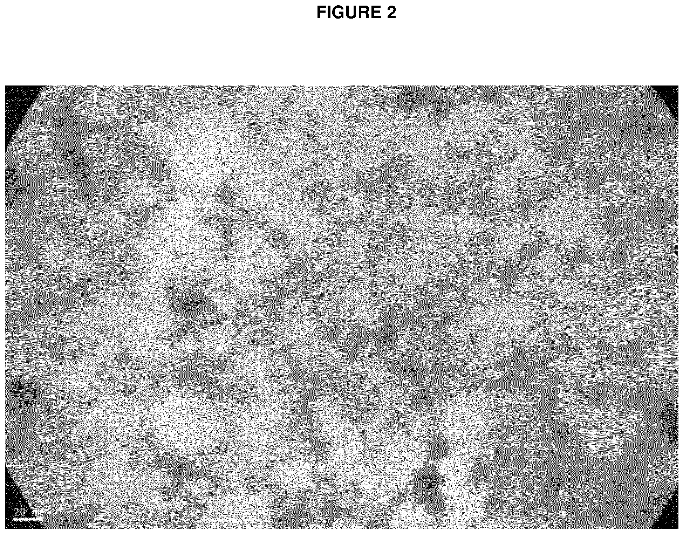

Structured nanoparticles loaded with nifurtimox. Transmission electron microscopy (TEM) performed at the 1st Chair of Histology of the Faculty of Medicine of the UBA on October 29, 2012, with a contrast obtained with a 0.1% Osmium Tetroxide solution in a buffer of phosphates, according to a technique developed ad hoc. 400,000 X magnification. Nanoparticles have a size of approximately 1 nm and can be visualized as very abundant small rounded objects (SEE FIGURE 2).

### □ 2. Photomicrograph B) Scanning Electron Microscope (SEM)

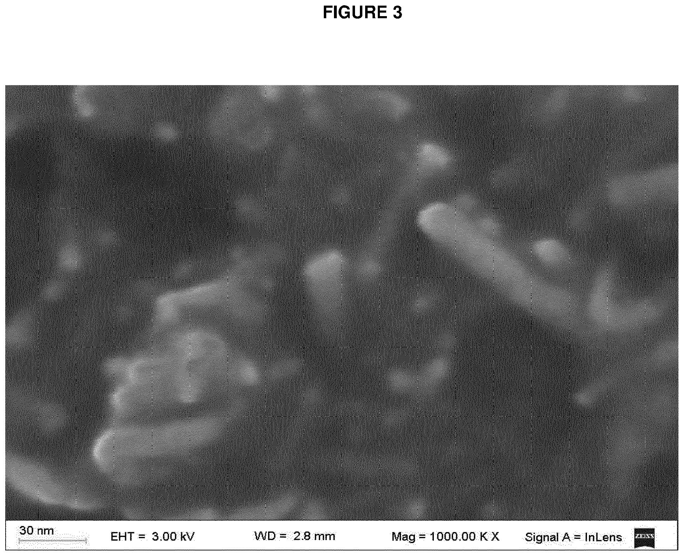

Scanning Electron Microscopy (SEM) carried out at the School of Exact Sciences of the University of Buenos Aires in 2016. Cylindrical agglomerates of NLC of approximately 20 nm in diameter can be observed. The resolution of the technique prevents separating the individual nanoparticles. (SEE FIGURE 3).

### □ 3. Photomicrograph C) atomic force microscope (AFM).

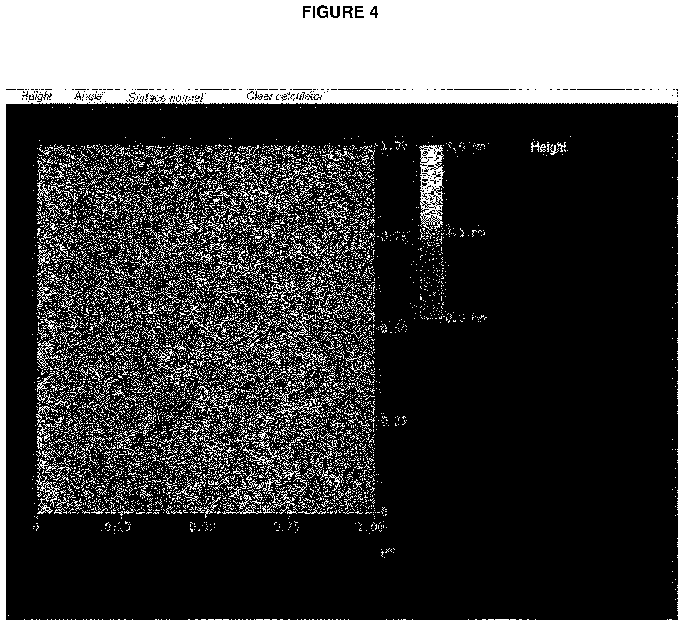

Atomic Force Microscopy showing the height of the NLCs prepared at CIN-Nanof. It is observed that the particles are homogeneous, spheriform and appear to be about 2 nm in height. Microscopy Laboratory of the School of Exact Sciences of the University of Buenos Aires in 2016 (SEE FIGURE 4)

### 4) EXAMPLES OF REALIZATION

The conventional method for the preparation of NLC is high pressure homogenization (HPH). The preparation of NLC with HPH includes several critical process conditions, such as high temperature, high pressure, and high surfactant concentration. The high concentration of the surfactant turns out to be particularly problematic [32]. That is why it was decided to use the "solvent diffusion" preparation method.

### • Extraction of Nifurtimox (NFX) from tablets and spectrophotometric identification

NFX tablets with the fantasy name of "Lampit" were used, prepared in the Republic of EI Salvador and registered in the Republic of Guatemala, from lot 09060093, with an expiration date in June 2014, containing 120 mg of NFX each and an average weight of 400 mg, were pulverized and subjected to the extraction of the active ingredient, through immersion in 10 ml of acetone per tablet, filtering and drying at 60 ° C in a porcelain crystallizer, repeating the operation with absolute ethanol. A powder was obtained consisting of acicular yellow crystals, practically insoluble in water, with a yield of approximately 1 gram per 10 tablets. The mentioned powder contained a 98.7% average nifurtimox, in a spectrophotometric measurement against a primary standard of NFX from Sigma-Ehrlich at 401 nm wavelength.

### • Preparation of nanoparticles

Following the solvent diffusion technique [32], 10 mg of NFX were dissolved in a mixture in equal proportions of absolute ethanol and acetone, adding palmitic acid (PA) and linoleic acid (LA) to form a 30/70% mixture of both lipids. Each sample was heated in a water bath at 70 ° C until complete dissolution, to finally diffuse under mechanical stirring in 120 ml of water at 70 ° C. Subsequently, half of the samples were sonicated for 15 minutes. Once obtained, the nanoparticles were flocculated by adding 1 N hydrochloric acid to pH 1.2 (around 13 ml per sample).

### • Treatment of the aqueous phase

The flocculated mixture, with an intense yellow color and a certain turbidity, was subjected to a filtration through medium-grain paper, obtaining a clear, yellow liquid, pH 1.2, stable at room temperature. Subjected to spectrophotometric measurement it turned out to contain almost all of the NFX introduced into the system. Taken to neutrality with drops of 1N sodium bicarbonate, it was dried in a porcelain crystallizer at 40 ° C for 48 hours. A yellowish brown amorphous powder was obtained, which was instantly re-suspended in the presence of distilled water, generating a clear, non-opalescent, intensely yellow liquid that absorbs light at a wavelength of 401 nm. There were no differences between the sonicated and non-sonicated samples.

### • Study of the suspension to the Z-Sizer: Z potential and average diameter

The macroscopically stable and clear liquid, filtered through a Teflon sieve with pores of 220 nm in diameter, was subjected to the examination of the Z potential, using the Z-sizer, which turned out to be +8 mV. In the measurement of the average size of the suspended particles, a marked unimodal distribution of same was obtained in the approximate size of 0.95 nanometers. There were no differences between the sonicated and non-sonicated samples.

### • Examination of the particles by transmission electron microscopy (TEM)

The images obtained by means of TEM with a magnification of 400,000 X, show homogeneous particles, of around 1 nanometer, approximately spherical. The contrast was obtained with a 0.1% Osmium Tetroxide solution in a phosphate buffer, a technique adapted ad hoc. (Exam carried out at the 1st Chair of Histology, Faculty of Medicine, University of Buenos Aires). There were no differences between the sonicated and non-sonicated samples.

### • Examination of the particles by atomic force microscopy

The images obtained show spheroid particles of approximately 1 to 2 nm in diameter, isolated or arranged in prisms of approximately 200 nm thick and varying lengths. There were no differences between the sonicated and non-sonicated samples.

### • Titration of the NFX present in the suspension and in the particles

The NFX present in the colloidal suspension and in the particles was titrated [37] by dissolving a dried sample of the suspension in acetone and recording the absorbance at 401 nm in a spectrophotometer, against a control prepared with the Sigma-Ehrlich primary standard, obtaining a concentration of 0.09 mg / ml of nifurtimox in the colloidal suspension and of approximately 4.7% p / p in the dried particles. There were no differences between the sonicated and non-sonicated samples.

### • Preparation of a NFX concentrated suspension

In order to maintain the experimental conditions, the initial stage of the preparation of the concentrated suspension repeated the technique described above using 30 times greater amounts of all the reagents. The suspension at pH 1.2 was filtered directly through medium grain paper, neutralized with 1 N sodium bicarbonate, dried at 40 ° C and dispersed again in distilled water at a concentration of 6 mg / ml of NFX, confirmed spectrophotometrically as described above.

### • Preparation of the concentrated drinkable suspension

83.3 ml of the suspension obtained, 10 ml of glycerin, 0.6 g of hydroxyethyl cellulose 10000 Cp, 3 ml of propylene glycol, 0.04 g of saccharin, 0.125 g of methyl paraben, 0.2 ml of vanilla essence, 1 ml of 1% solution of sunset yellow colorant and 2.5 ml of distilled water was provided. The methyl paraben and propylene glycol were placed in an erlenmeyer flask of adequate capacity and heated until completely dissolved. In another container the glycerin was incorporated and the hydroxyethylcellulose was dispersed, mixing until obtaining a homogeneous dispersion, with total absence of lumps; then the essence and the colorant were added to this dispersion, reserving this viscous preparation for the final mixture. Then, in a stainless steel pot, all of the 6 mg / ml NFX colloidal dispersion was introduced and the methyl paraben / propylene glycol solution was added, stirring constantly until homogeneous. The saccharin was dissolved in all of the distilled water and was incorporated into this homogeneous mixture. Finally, the dispersion of hydroxyethylcellulose in glycerin, essence and colorant was added, and the mixture was processed for half an hour in a colloid mill.

### • Titration of the concentrated drinkable suspension

8 ml of the concentrated drinkable suspension prepared with 12 ml of acetone were shaken in a decanting flask, producing the extraction of the NFX which was then titrated as described in 1.6. The concentration obtained was 4.8 mg / ml of the active ingredient.

### • Description and Stability

The suspension obtained is homogeneous, limpid, slightly opalescent, with a syrupy consistency, a vanilla aroma, a sweet taste and an intense yellow color. It has been macroscopically stable for a year since its preparation, both at room temperature and refrigerated at 4 ° C.

### • Preparation of NLC with different fatty acids

All NLCs were prepared by the exposed technique, generating in all cases, an aqueous colloidal suspension of nanoparticles, with the concentration of 10 mg of NFX (drug at 98% purity) per 100 ml of water (that is, 10% mg / ml NFX equivalent).

### • Fatty acids used

The following fatty acids were used:
Solid lipids at room temperature (between 19 degrees Celsius and 21 degrees Celsius, preferably at 20 degrees Celsius):
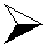 Stearic acid
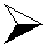 Palmitic acid
Liquid lipids at room temperature (between 19 degrees Celsius and 21 degrees Celsius, preferably at 20 degrees Celsius):
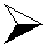 Oleic acid
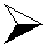 Linoleic acid
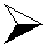 Palmitoleic acid
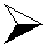 Arachidonic acid

The following nifurtimox carrier nanoparticle preparations were made, combining in all cases solid fatty acids with liquid fatty acids:
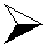 □ Stearic / oleic
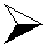 Stearic / linoleic
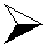 Stearic / palmitoleic
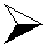 Stearic / arachidonic
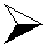 Palmitic / oleic
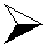 Palmitic / linoleic
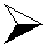 Palmitic / palmitoleic
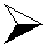 Palmitic / arachidonic

### • Result of preparations

In all cases, yellow suspensions without turbidity that were brought to an approximate pH of 6.0 with 1 N NaOH and then filtered through a 220 nanometer pore diameter Teflon filter.

### • Measurement of the zeta potential of the particles

This indicator measures the attraction or repulsion between the particles. In principle, the presence of a positive or negative charge greater than 30 millivolts generates a strong repulsion between the particles and this stabilizes the suspension. If the load is lower or null, close to the isoelectric point, it is assumed that it does not favor the stability of the suspension. However, a Z potential greater than approximately 30mV is not the only stability factor. There are other variables that influence the potential stability of the suspension, especially the size of the particles. A large size generates instability, while a small granulometry tends to stabilize the suspension. The final result depends on the balance between the independent variables.

**Table 5: Measurement results: As can be seen, the eight samples exhibit an electrical potential much less than (+ or -) 30 mV, which is considered an indicator of stability. The one that is furthest from the isoelectric point (0.0 mV) is the carrier of stearic / linoleic acids, which, however, with + 16.5 mV is still well below the limit that would indicate a predisposition to stability from the electrical charge. Conclusions: a) the Z potential of the studied particles cannot be considered as the exclusive criterion of choice for any of the samples. b) The absence of turbidity for one year and the macroscopic stability of the suspensions cannot be attributed to a high Z potential, the extremely small character of the NLC loaded with nifurtimox being examined constitutes an explanatory hypothesis.**

| FATTY ACID COMBINATION | "Z" POTENTIAL |
|---|---|
| STERIC / OLEIC ACIDS | (+) 8,0 |
| STERIC / LINOLEIC ACIDS | (+) 16,5 |
| STERIC / PALMITOLEIC ACIDS | (+) 5,0 |
| STERIC / ARAACHIDONIC ACIDS | (+) 3,0 |
| PALMIC / OLEIC ACIDS | (+) 1,4 |
| PALMITIC / LINOLEIC ACIDS | (-)0,2 |
| PALMITIC / PALMITOLEIC ACIDS | (+)5,1 |
| PALMITIC / ARAACHIDONIC ACIDS | (+) 0,9 |

### • Determination of size

The granulometry can be carried out by the criterion of volume% and / or by the intensity of the light%.

**Table 6: Size by Volume%. Above all, a very low particle size is observed, ranging between 0.69 and 3.14 nanometers, which is unusual for these nanoparticles, which in other studies frequently exhibit more than 100 nm in average diameter. Another aspect is the unimodal nature of the distribution since only a single peak is observed for each type of fatty acid combination, and that peak concentrates 100% of the population. The examination of the graphical representation of Volume% versus size in nm (not shown here), shows that in the combination of Palmitic / Oleic acids ONLY, of which 4 successive determinations were made, a dispersive bias is observed in the results that takes away the precision of the 3.14 nanometer data, since some measurements give peaks of approximately 1 and 8 nm. In the rest of the observations, the readings for each combination of fatty acids were made from 4 to 6 times and showed high precision.**

| FATTY ACID COMBINATION | nanometers |
|---|---|
| STERIC / OLEIC ACIDS | 0,91 |
| STERIC / LINOLEIC ACIDS | 0,72 |
| STERIC / PALMITOLEIC ACIDS | 3,01 |
| STERIC / ARAACHIDONIC ACID | 1,3 |
| PALMIC / OLEIC ACIDS | 3,14 |
| PALMITIC / LINOLEIC ACIDS | 0,69 |
| PALMITIC / PALMITOLEIC ACIDS | 0,73 |
| PALMITIC / ARAACHIDONIC ACIDS | 1,5 |

This modality is considered as more significant than Volume% and offers more information.

**Table 7: Granulometric study by light intensity%. It is observed that the size distribution is polymodal. In the case of the stearic / oleic combination, almost 60% of the particle size exceeds 10,000 nanometers. In the case of the stearic / arachidonic combination, almost 80% of the particle size exceeds 5000 nanometers. In the palmitic / arachidonic combination, almost 70% of the particle size exceeds 7500 nanometers. These particulate segments are obviously not nanometric. In the stearic / linoleic combination, almost 70% of the particles exceed 300 nanometers. In the palmitic / palmitoleic combination, more than 60% of the particles exceed 400 nanometers. Given that all the suspensions had been filtered by a pore smaller than 220 nm 24 hours before the measurement, it is concluded that in a few hours the particles of the five suspensions mentioned suffered strong agglutination with a diameter greater than the mentioned pore, a process that, if continued in over time, could destabilize the suspension.**

| FATTY ACID COMBINATION | 1° peak, nm | 1° peak, % | 2° peak, nm | 2° peak, % | 3° peak, nm | 3° peak, % | Total % |
|---|---|---|---|---|---|---|---|
| STERIC / OLEIC ACIDS | 0,91 | 40,1 | 0 | 0 | >10000 | 59,9 | 100 |
| STERIC / LINOLEIC ACIDS | 0,77 | 17,1 | 5,57 | 14,3 | 307,8 | 68,6 | 100 |
| STERIC / PALMITOLEIC ACIDS | 3,26 | 19,5 | 147,5 | 80,5 | 0 | 0 | 100 |
| STERIC / ARAACHIDONIC ACIDS | 0,95 | 20,8 | 0 | 0 | >5000 | 79,2 | 00 |
| | 3,39 | 17,7 | 22,7 | 32,5 | 205,1 | 49,8 | 100 |
| PALMIC / OLEIC ACIDS | 0,69 | 30,6 | 6,39 | 16,2 | 183,3 | 53,2 | 100 |
| PALMITIC / LINOLEIC | 0,74 | 16,7 | 202,4 | 23,2 | 422,3 | 60,1 | 100 |
| ACIDS | | | | | | | |
| PALMITIC / PALMITOLEIC ACIDS | 0,82 | 31,3 | 0 | 0 | >7500 | 68,7 | 100 |

The distribution of the combination of stearic and palmitoleic acids shows a bimodal distribution, with a 20/80% ratio of the sizes of slightly more than 3 and slightly more than 147 nanometers. Given that the volume% distribution offers a single peak of just over 3 nanometers in this combination, lets remember that with the low Z potential of +5 (which facilitates clumping), the 147 nanometer particles are likely to form clumps in the smaller particles, thermodynamically facilitated. The polydispersity index (PDI, not shown in the table, see below) is in this stearic / palmitoleic combination greater than 0.8 (stability is considered to improve below PDI <0.5), which predisposes to mentioned hypothesis of a weak clumping around the 147 nanometer peak.

The combination of palmitic / oleic acids is trimodal for the variable size / intensity%. Half of the nanoparticles are concentrated in a particle size of 205 nanometers. Almost a third have an average diameter of 23 nanometers. Slightly less than a fifth (18%) shows a size of just over 3 nanometers, practically identical to the data obtained with the "Volume%" method. The polydispersity index (PDI) of this combination is 0.6 (not shown in the table, see below), higher than the PDI index of 0.5 which is considered the limit of stability, as mentioned earlier. Therefore, the hypothesis of the agglutination of smaller particles around two thermodynamically facilitated grouping sizes, of approximately 23 and 205 nanometers, fits here.

Finally, the combination of palmitic / linoleic acids also offers a trimodal size configuration. More than half of the particles are grouped at 183 nanometers; almost a third of the objects are less than 0.7 nanometers (the same as determined by the volume% method) and the rest of the particles are just over 6 nanometers. A Polydispersity Index (PDI) greater than 0.9 (not shown in the table, see below) leads us to think, even more than in the two previous cases, that the nanoparticles of the two largest peaks are affected by a tendency to clumping. This is facilitated by the almost zero Z potential of - 0.23 (see above), which facilitates this process as it does not interfere at all with the adherence of the nanoparticles to each other.

**Table 8 Polydispersity Index (PDI) for Light intensity%**

| FATTY ACID COMBINATION | **PDI** |
|---|---|
| STERIC / OLEIC ACIDS | 0,9 |
| STERIC / LINOLEIC ACIDS | 0,7 |
| STERIC / PALMITOLEIC ACIDS | 0,8 |
| STERIC / ARAACHIDONIC ACIDS | 0,8 |
| PALMIC / OLEIC ACIDS | 0,6 |
| PALMITIC / LINOLEIC ACIDS | 1 |
| PALMITIC / PALMITOLEIC ACIDS | 0,8 |
| PALMITIC / ARAACHIDONIC ACIDS | 0,9 |

### • Functional effectiveness of nifurtimox nanoparticles in the in vitro treatment of Chagas disease

Very significant differences in antiparasitic effectiveness were observed between nifurtimox as such and nifurtimox in nanostructured nanoparticles in in vitro models. Nifurtimox nanoparticles have a trypanocidal effect on trypomastigotes and on amastigotes. The use of nano-structured nanoparticles of Nifurtimox has been almost two orders of magnitude more potent than Nifurtimox as such on trypanosomes and three orders of magnitude more potent than Nifurtimox as such on amastigotes.

### • Trials on blood trypomastigotes

Blood trypomastigotes of strain RA were purified from mouse blood during the peak of parasitaemia. The maintenance of the strain is usually carried out by successive weekly passages in CF1 mice, 21-day-old males, infected with 10⁵ trypomastigotes via IP.
- Purification of trypomastigotes: 5 volumes of PBS 3% fetal bovine serum were added to the blood obtained from the infected mice. Erythrocytes were separated by low speed sedimentation and incubated for 1 hour at 37 ° C. Finally, the supernatant was centrifuged at 10,000 xg for 30 minutes in order to obtain a high concentration of blood trypomastigotes for in vitro tests.
- Trypanocidal effect of drugs: Incubation of 2.4 x 10⁵ trypomastigotes / well (100 µL) in RPMI 5% SFB in triplicate in 96-well plates with 100 µL of the different concentrations of nifurtimox and nanoparticles of nifurtimox in a final range of 0.3125 to 10 µg / ml for 24 hours at 37 ° C and 5% CO2.
- Measurement of trypanocidal activity: count in Neubauer chamber considering 100% viability to the average count obtained for the controls without drug From the analysis of the figures below it is evidenced that two to three times lower doses of nanoparticles of nifurtimox were needed to reach 50% lethality (0.57 ± 0.01 vs 1.45 ± 0.46).

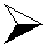 Nanoparticles of nifurtimox on trypomastigotes:

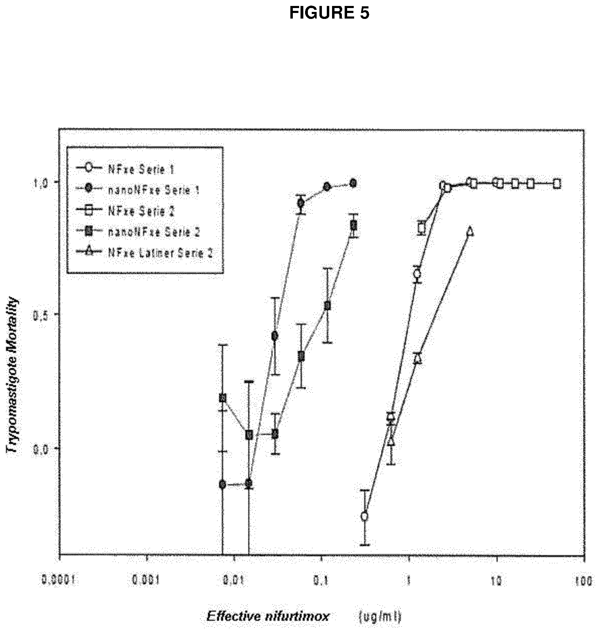

Dose response curves are shown for drug nifurtimox as such and nanoparticles of nifurtimox on the survival of trypomastigotes (SEE FIGURE 5).

### • Essays on amastigotes

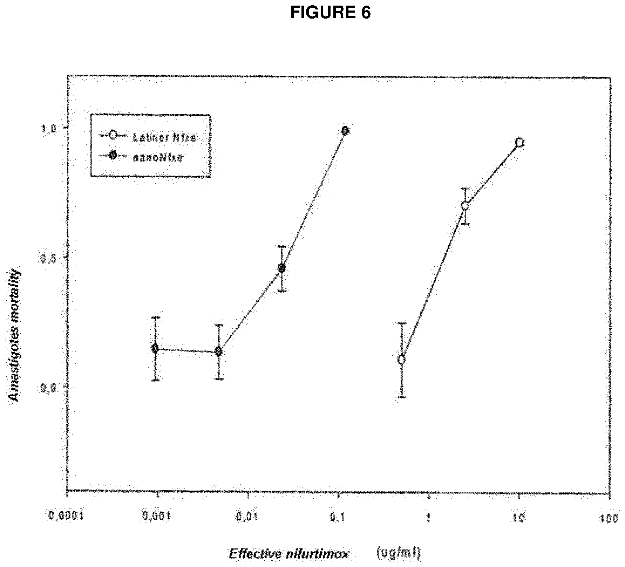

In vitro effectiveness of nifurtimox nanoparticles on apoptosis of tumor cells of the central nervous system: astrocytomas and retinoblastomas (SEE FIGURE 6).

Effect of nifurtimox nanoparticles on the viability of astrocytoma cells in culture. The cytocidal effect of nifurtimox nanoparticles on Astrocytoma cells in culture can be observed. It is interesting to note that GL26 NT cell lines are from rat neural tumor, while spherical and adherent MSP12, spherical and adherent MGG8 lines are human in culture. From 500 mM inhibitory activity can be observed in all exposed cells (SEE FIGURE 6).

Cell infection: 5 x 104 Vero / well cells were seeded on glass slides in 24-well plates for 24 hours at 37 ° C and 5% CO2. Then they were incubated with blood trypomastigotes of the RA strain at a rate of 5 parasites / cell (MOI: 5) for 3 hours.

Effect of nanoparticles with Nftx on amastigotes of T. cruzi: Once the infection was completed, the cells were washed in order to eliminate free parasites and incubated with Nftx (0.02-10 µg / ml) and nano Nftx (0.02-2.5 µg / ml) in triplicate for 72 hours at 37ºC and 5% CO2. Finally, they were fixed with methanol for 5 minutes and stained with Giemsa. For the purpose of measuring the parasite load per cell, 20 infected cells were randomly photographed for each drug concentration tested.

The number of amastigotes / cell was determined by image analysis using Image J software.

We consider that the nanoparticulation process of nifurtimox allows an increase in the transmembrane passage and an increased intracellular bioavailability of the drug, which gives nifurtimox a new and fundamental quality for therapeutic objectives, namely: high efficacy on intracellular amastigote nests, which could eventually become an effective therapy for Chagas disease in the chronic phase, today intractable.

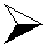 Nifurtimox nanoparticles on amastigotes:

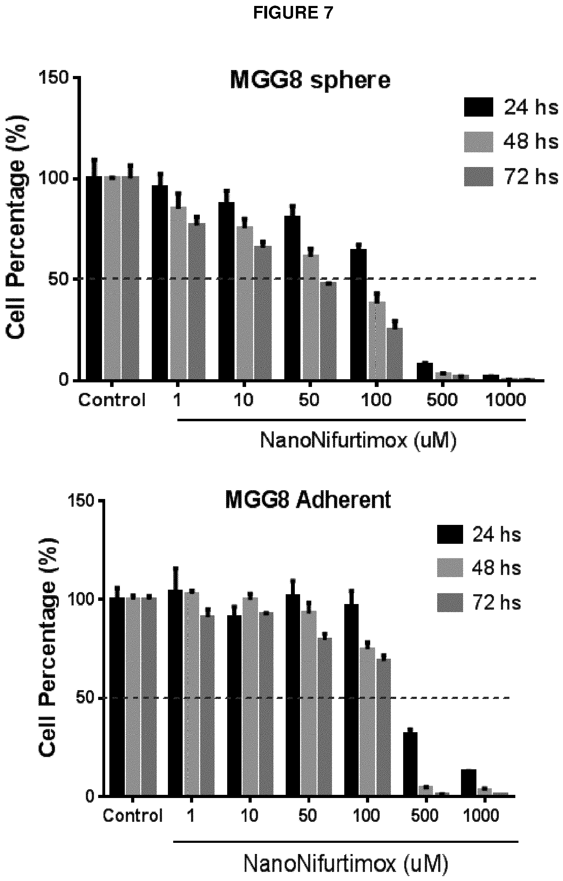

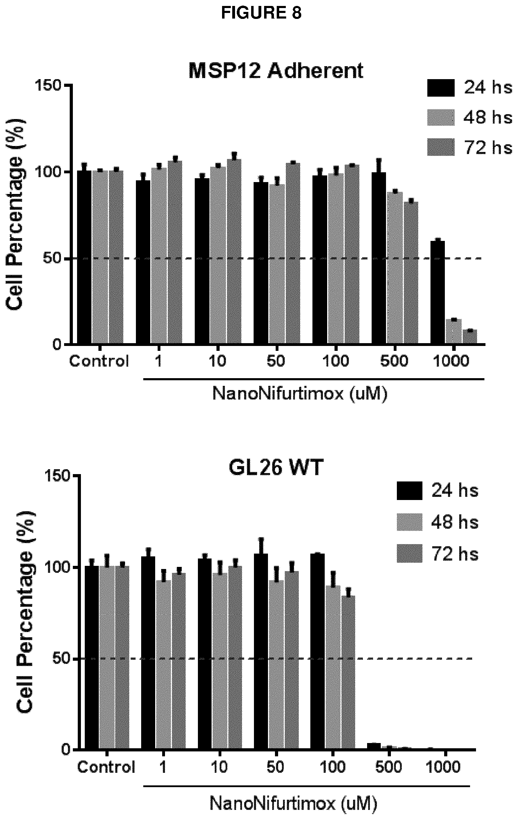

Dose response curves are shown for drug nifurtimox as such and nanoparticles of nifurtimox on the survival of amastigotes (SEE FIGURES 7 and 8).

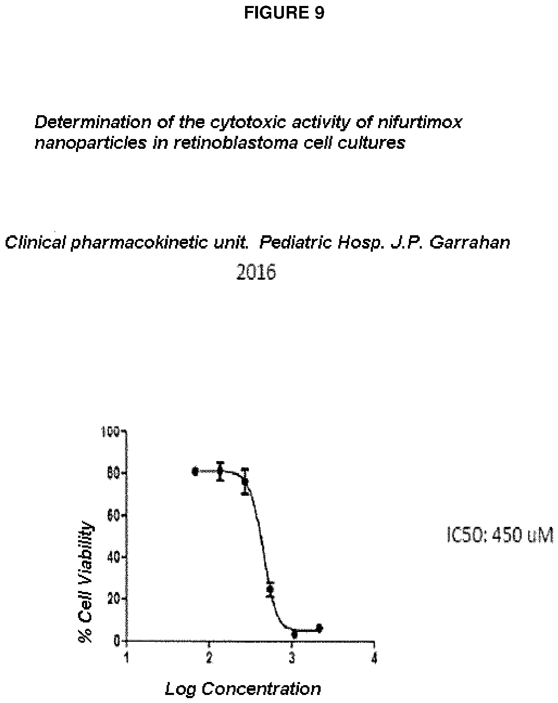

In FIGURE 9, the effect of nanoparticles of nifurtimox on the viability of etinoblastoma cells in culture is observed. The in vitro antineoplastic activity of nifurtimox nanoparticles is observed on Retinoblastoma cells in culture. The cytotoxicity assay was performed on commercial Y79 retinoblastoma cell lines. They were seeded at a rate of 20,000 cells per well in 96-well plates. Cell proliferation was measured by the MTT colorimetric method.

Also, the inventors emphasize that the composition of the invention has the following relevant characteristics that deserve to be commented, they are:
- Its administration is oral, pulmonary, intravenous, rectal, ophthalmic, colonic, parenteral, intracisternal, intrathecal, intravaginal, intraperitoneal, otic, local, transdermal, buccal, nasal and topical.
- Transdermal penetration enhancers (enhancers) from the family of sulfoxides are used, especially those called dimethylsufoxide (DMSO), dimethylacetamide (DMAC) and dimethylformamide (DMF), from the family of oxazolidones of ureas, azones and pyrrolidones, from the family of essential oils such as terpenes and terpenoids and from the family of fatty acids
- The dosage form is selected from among liquid dispersions, gels, aerosols, ointments, creams, lyophilized forms, tablets, orodispersible tablets, capsules, colloidal suspensions, dissolvable papers and jet (needle free).
- The dosage form is selected from immediate release, controlled release, delayed release, prolonged release, pulsed release formulations, or combinations thereof.
- It comprises one or more pharmaceutically stable excipients, vehicles or combinations thereof.
- The additional active ingredient is selected from the group of diuretics, α-blockers, β-blockers, ACE inhibitors, calcium channel blockers, angiotensin antagonists, nervous system inhibitors and vasodilators.

### BIBLIOGRAPHIC REFERENCES CITED IN THE SPECIFICATIONS

[1] **Pan American Health Organization (PAHO). Quantitative estimation of Chagas disease in the Americas. Montevideo,** OPS/HDM/CD/425, 2006.
[2] J.C. Rodrigues Coura and S. L. de Castro. "A Critical Review on Chagas Disease Chemoterapy". Memories of Instituto Oswaldo Cruz, vol. 97, no. 1, pp. 3-24, 2002.
[3] A.M. Strosberg, K. Barrio, V.H. Stinger, J. Tashker, J.C. Wilbur, L. Wilson, and K. Woo. Chagas disease: a Latin American nemesis, 2007.
[4] C.M. Morel. "Reaching maturity- 25 years of the TDR". Parasitology Today, vol. 16, pp. 522-525, 2000.
[5] A. Moncayo. "Chagas disease: current epidemiological trends after the interruption of vectorial and transfusional transmission in the Southern Cone countries". Memorias del Instituto Oswaldo Cruz, vol. 98, pp. 577-591, 2003.
[6] D.A. Leiby, J.R.M. Herron, E.J. Read, B.A. Lenes, and R.J. Stumpf. "Trypanosoma cruzi in Los Angeles and Miami blood donors: impact of evolving donor demographics on seroprevalence and implications for transfusion transmission". Transfussion, vol. 42, pp. 549-55, 2002.
[7] C.B. Beard, G. Pye, F.J. Steurer, R. Rodriguez, R. Campman, and A.T. Peterson. "Chagas disease in a domestic transmission cycle, southern Texas, USA". Emergency Infection Disesase, vol. 9, pp. 103-105, 2003.
[8] J.H. Díaz. "Recognizing and reducing the risks of Chagas disease (American trypanosomiasis) in travelers". Journal Traveller Medicine, vol. 15, pp.184-195, 2008.
[9] A. Prata. "Clinical and epidemiological aspects of Chagas' disease". Lancet Infection Disease, vol. 2, pp. 92-100, 2001.
[10] J. A. Urbina and R. Docampo. "Specific Chemoterapy of Chagas disease: controversies and advances". Trends Parasitology, vol. 93, pp.203-214, 2003.
[11] G.C. Levi, V. Amato, J.F. Neto, and D. Araujo. "Analise de manifestacoes colaterais devidas ao uso do medicamento Ro-7-1051 nitromidazolico especifico da doenca de Chagas". Revista del Instituto de Medicina Tropical de Sao Paulo, vol. 17, pp.49-52, 1975.
[12] A. L. de Andrade, F. Zicker, R.M. de Oliveira, S. Almeida Silva, A. Luquetti, L.R. Travassos, I.C. Almeida, S.S. de Andrade, J.G. de Andrade, and C.M. Martinelli. "Randomised trial of efficacy of benznidazole in treatment of early Trypanosoma cruzi infection". Lancet, vol. 348, pp.1407-1413, 1996.
[13] S. Sosa Estani, E.L. Segura, A.M. Ruiz, E. Velazquez, B.M. Porcel, and C. Yamporis. "Efficacy of chemoterapy with benznidazole in children in the indeterminate phase of Chagas disease". American Journal of Tropical Medical Hygiene, Vol. 59, pp. 526-529, 1998.
[14] J.R. Cancado. "Criteria of Chagas disease cure". Memories Instituto Oswaldo Cruz, vol. 94, pp. 331-336, 1999.
[15] S. Sosa Estani and E.L. Segura. "Treatment of Trypanosoma cruzi infection in the undeterminated phase. Experience and current guidelines in Argentina". Memorias del Institute Oswaldo Cruz, vol. 94, pp.363-365, 1999.
[16] S. Murta, R. Gazzinelli, Z. Brener, and A. Romanha. "Molecular characterization of susceptible and naturally resistant strains of Trypanosoma cruzi to benznidazole and nifurtimox". Molecular Biochemical Parasitology, vol. 93, pp. 203-214,1998.
[17] C. Breyrer, J.C. Villar, V. Suwanvanichkij, S. Singh, S. Baral, and E.J. Mills. "Neglected diseases, civil conflicts, and the right to health". Lancet, vol. 370 pp. 619-627, 2006.
[18] P. Trouiller, P. Olliaro, E. Torreele, J. Orbinski, R. Laing, N. Ford. "Drug development for neglected diseases: a deficient market and a public-health policy failure". Lancet vol. 359, pp. 2188-2194, 2002.
[19] M.S. Braga, L. Lauria-Pires, E.R. Argañaraz, R.J. Nascimento, and A.R.L. Texeira. Magazzine of the Instituto de Medicina Tropical de Sao Paulo, vol. 42, no. 3, May/June, 2000.
[20] R. do Campo and S.N.J. Moreno. Fed. Proceedings, vol. 45, pp. 2471-2476, 1986.
[21] **WHO/Make medicines child size.** Available at: http://www.who.int/childmedicines/en (accesed 07/2009. Ref. D.A. Chiapetta, C. Hocht and A. Sosnik "A Higly Concentrated and Taste-Improved Aqueus Fromulation of Efavirenz for a More Appropiate Pediatric Management of the Anti-HIV Therapy", Current HIV Research, vol. 8, pp. 223-231, 2010.
[22] J.F. Standing and C. Tuleu. "Paediatric formulations-getting to the heart of the problem". International Journal Pharmaceutical, vol. 300, pp. 56-66,2005.
[23] I. Choonara, and S. Conroy. "Unlicencied and off-label drug use in children. Implications for safety". Drug Safety, vol. 25, pp. 1-5, 2002.
[24] T. Nunn and J. Williams. "Formulations of medicines for children". British journal of Clinical Pharmacology, vol. 59, pp. 674-676, 2005.
[25] T. Eileen Kairuz, D. Gargiulo, C. Bunt, and S. Garg. "Quality, safety and efficacy in the "off-label' use of medicines". Current Drug Safety, vol 2, pp. 89-95, 2007.
[26] E.L. Romero and M.J. Morilla, "Nanotechnological approaches against Chagas disease". Advanced Drug Delivery Reviews, doi:10.1016/addr.2009.11.025, 2009.
[27] P. Severino, T. Andreani, A.S. Macedo, J.F. Fangueiro, M.H.A. Santana, A.M. Silva, and E.B. Souto. "Current State-of-Art and New Trends on Lipid Nanoparticles (SLN and NLC) for Oral Drug delivery" Hindawi Publishing Corporation. Journal of Drug Delivery. Vol. 2012. Article ID 750891, 10 pages. Doi:10.1155/2012/750891, 2012.
[28] M. Radke, E.B. Souto and R.H. Muller. "Nanostructured Lipid Carriers: A Novel Generation of Solid Lipid Drug Carriers". Pharmaceutical Technology Europe, vol. 17, no. 4, pp. 45-50, 2005.
[29] Y. Kawashima, H. Yamamoto, H. Takeuchi, T. Hino, and T. Niwa. "Properties of a peptide containing DL-lactide/glycolide copolymer nanospheres prepared by novel emulsion solvent diffusion method". European Journal of Pharmaceutics and Biopharmaceutics, vol. 45, pp. 41-48, 1998.
[30] F.Q. Hu, H. Yuan, H.H. Zhang, and M. Fang. "Preparation of solid lipid nanoparticles with clobetasol propionate by a novel solvent diffusion method in aqueus system and physicochemical characterization". International Journal of Pharmaceutics, vol. 239, pp. 121-128, 2002.
[31] F.Q. Hu, Y. Hong, and H. Yuan. "Preparation and characterization of solid lipid nanoparticles containing peptide". International Journal of Pharmaceutics, vol. 273, pp. 29-35, 2004.
[32] F.Q. Hu, S.P. Jiang, Y.Z. Du, H. Yuan, Y.Q. Ye, and S. Zeng. Preparation and characterization of stearic acid nanostructured lipid carriers by solvent diffusion method in an aqueus system". Coloids and Surfaces B: Biointerfaces. Vol 45, pp. 167-173, 2005.
[33] R.H. Muller, K. Mader, and S. Gohla. "Solid Lipid Nanoparticles (SLN) for controlled drug delivery - a review of the state of the art". European Journal of Pharmaceutics and Biopharmaceutics, vol. 50, pp. 161-177, 2000.
[34] R.H. Muller, S. Maaben, H. Weyhers, F. Specht, and J.S. Lucks. "Cytotoxicity of magnetite loaded polylactide, polylactide/glycolide particles and solid lipid nanoparticles (SLN)". International Journal of Pharmaceutics, vol. 138, pp. 85-94, 1996.
[35] R.H. Muller, D. Ruhl, and S.A. Runge. "Biodegradation of solid lipid nanoparticles as a function of lipase incubation time". International Journal of Pharmaceutics, vol. 144, pp. 115-121, 1996.
[36] A. Moncayo. "Chagas disease: current epidemiological trends after the interruption of vectorial and transfusional transmission in the Southern Cone countries". Memorias del Instituto Oswaldo Cruz, vol. 98, pp. 577-591, 2003.
[37] Sanchez G, Cuellar D, Zulantay I, Gajardo M, González-Martin G. Cytotoxicity and trypanocidal activity of nifurtimox encapsulated in ethylcyanoacrylate nanoparticles. Biological Research v.35, n.1, Santiago 2002.
[38] Saulnier Sholler GL, Brard L, Straub JA, Dorf L, Illyene S, Koto K, Kalkunte S, Bosenberg M, Ashikaga T, Nishi R. Nifurtimox induces apoptosis of neuroblastoma cells in vitro and in vivo. J Pediatr Hematol Oncol. 31(3) pp 187-193. 2009*.*
[39] Cabanillas Stanchi KM, Bruchelt G, Handgretinger R, Holzer U. Nifurtimox reduces N-Myc expression and aerobic glycolysis in neuroblastoma. Cancer Biol Ther. 2015;16(9):1353-63.

## Claims

1. Pharmaceutical composition with antineoplastic and antitumor action, **characterized in that** it comprises structured lipid nanoparticles that have a size between 0.1 nm and 100 nm in diameter, with spheroidal shape and containing particles of one or more active ingredients for the treatment of diseases caused by trypanosomes and for the treatment of tumors of neural origin, and at least one coupling of two fatty acids formed by a saturated fatty acid or semisolid lipid at a temperature between 19ºC and 21ºC and an unsaturated or liquid fatty acid at a temperature between 19ºC and 21ºC.

2. Pharmaceutical composition with antineoplastic and antitumor action, according to claim 1, **characterized in that** said coupling of two saturated and unsaturated acids have a formation temperature of preferably 20ºC.

3. Pharmaceutical composition according to claim 1, **characterized in that** said active ingredient for trypanosomiasis is selected from benzimidazole, nifurtimox, ethanidazole, buthionine sulfoximine, nitroimidazole derivatives, eflornithine, melarsoprol, pentamidine, surimin and fexinidazole.

4. Pharmaceutical composition, according to claim 3, **characterized in that** said active ingredient for trypanosomiasis is selected from benznidazole, nifurtimox or the combination of benznidazole nanoparticles and nifurtimox nanoparticles.

5. Pharmaceutical composition, according to claim 1, **characterized in that** the saturated fatty acid or semisolid lipid is selected from stearic acid and palmitic acid, and the unsaturated fatty acid or liquid lipid is selected from oleic acid, arachidonic acid, palmitoleic acid, alpha-linoleic acid, or gamma-linoleic acid.

6. Pharmaceutical composition, according to claim 1, **characterized in that** said active ingredient is buthionine sulfoximine, for the treatment of tumors of neural origin, preferably pediatric tumors of neural origin.

7. Pharmaceutical composition, according to claim 1, **characterized in that** said coupling of two fatty acids comprises the saturated fatty acid palmitic acid.

8. Pharmaceutical composition, according to claim 1, **characterized in that** said coupling of two fatty acids comprises the saturated fatty acid stearic acid.

9. Pharmaceutical composition, according to claim 1, **characterized in that** said coupling of two fatty acids comprises the polyunsaturated fatty acid linoleic acid.

10. Pharmaceutical composition, according to claim 1, **characterized in that** said coupling of two fatty acids comprises the unsaturated fatty acid palmitoleic acid.

11. Pharmaceutical composition according to claim 1, **characterized in that** said structured lipid nanoparticles have a size preferably from 1 nm to 10 nm in diameter.

12. Pharmaceutical composition according to claim 11, **characterized in that** said structured lipid nanoparticles preferably have a size of 1 nm.

13. The pharmaceutical composition according to claim 1, **characterized in that** it is a medicine for the treatment of Chagas Mazza disease.

14. The pharmaceutical composition according to claim 1, **characterized in that** it is a medicine for the treatment of sleeping sickness (human African trypanosomiasis) produced by the parasite Trypanosoma brucei gambiense and transmitted by the vector (fly of the genus Glossina) tse- tsé or for the treatment of cutaneous or visceral leishmaniasis, which is a disease caused by the infection of several species of Leishmania, a protozoan parasite of the Trypanosomatidae family or other diseases caused by protozoa such as Toxoplasmosis, malaria and trichomoniasis. Toxoplasma: family Sarcocystidae; Malaria: Plasmodiidae family; Trichomonads: Trichomonadidae family.

15. The pharmaceutical composition, according to claim 1, **characterized in that** it is a drug for the treatment of tumor diseases of the central nervous system such as astrocytoma, retinoblastoma and neuroblastoma.

16. The pharmaceutical composition according to claim 1, **characterized in that** it is a drug where the active ingredient is nifurtimox associated with an antitumor drug of the cytostatic, hormonal or immunological type.

17. The pharmaceutical composition according to claim 1, **characterized in that** it is a medicine where the preparations of a cytostatic, hormonal or immunological nature are administered at lower doses than those currently indicated.

18. The pharmaceutical composition according to claim 1, **characterized in that** it is a medicine for the treatment of diseases and so-called rare or orphan tumors known as: ependymoma, neurofibromatosis type I and II, carcinoids, primitive neuroectodermal tumor, pheochromocytoma, neuroendocrine tumor, VIPoma

19. A method for obtaining said pharmaceutical composition according to claim 1, **characterized in that** it comprises the following steps:
a) Weigh from 140 to 160 mg, preferably 150 mg of active ingredient for trypanosomiasis, 1300 mg of solid saturated fatty acid at a temperature from 19 degrees centigrade to 21 degrees centigrade, preferably at 20 degrees centigrade (which will be called "room temperature"), and 440 mg of liquid unsaturated fatty acid at a temperature from 19 degrees centigrade to 21 degrees centigrade, preferably at 20 degrees centigrade, both of pharmacopoeial quality;
b) Measure from 55 ml to 65 ml, preferably 60 ml of analytical grade acetone, from 55 ml to 65 ml, preferably 60 ml of analytical grade absolute ethyl alcohol;
c) Mix the liquids from step b), in a 2000 ml borosilicate glass Erlenmeyer flask, and heat between 60.0 degrees centigrade to 80.0 degrees centigrade, preferably at 70.0 degrees centigrade;
d) Place the flask with the mixture of heated liquids from step c), on a heated magnetic stirrer, regulated at the same temperature, and add the active ingredient, the solid saturated fatty acid at room temperature and the liquid fatty acid at room temperature under stirring from 250 rpm to 300 rpm, preferably at 275 rpm, for 10 minutes to 20 minutes, preferably 15 minutes. A clear yellow liquid is obtained;
e) Without interrupting the stirring, add 1000 ml of double distilled water previously heated from 65 ° C to 75 ° C, preferably at 70 ° C, stirring and heating is maintained at the stipulated speed and temperature for 10 to 20 minutes, preferably 15 minutes and an intense yellow cloudy liquid is obtained,
f) Suspend heating and keep stirring at the stipulated speed, 60 ml of 1N Hydrochloric acid are added and stirring is maintained for 15 minutes;
g) The preparation of step f), let it rest until it reaches room temperature and filter with fine-grade filter paper, the liquid phase of the mixture offers a clear appearance of an intense yellow color and the precipitate obtained is little abundant, amorphous, white in color and the pH of the liquid is 2.0;
h) Place the liquid obtained in stage g), again under stirring at room temperature and add a 1N NaOH solution little by little in 5 ml portions. After incorporating each portion, the pH must be tested and the operation is suspended when the pH is 5.5;
i) Filter the mixture again through a fine-grained filter paper, the precipitate is very sparse, white in color and the liquid is completely clear and of an intense yellow color;
j) Filter the liquid through a Teflon filter with 220 nanometer pores and store it in clean sterile glass or PVC containers, in a cool place (it can be a refrigerator at 4ºC, but not a freezer) and protected from light; Y
k) Finally obtain the liquid that contains the nanoparticles of one or more active ingredients for trypanosomiasis in colloidal suspension, the Z potential ranges from -30 to +30, preferably +8, the particles measured by Z-sizer and ultramicroscopy have from 0,1 nanometers to 100 nanometers, preferably 1 to 10 nanometers, particularly 1 nanometer in diameter and with spheroidal shape.
